# EUROPEAN PATENT APPLICATION

(11) **EP 3 511 408 A1**
(43) Date of publication of application: **17.07.2019**
(21) Application number: 16915835.9
(22) Date of filing: 12.09.2016
(51) Int. Cl.: C12N 1/20, C12N 9/16, C12N 9/22, C12N 9/52, A61K 38/46, A61K 38/48, A61P 31/14, A61P 31/20

(54) **COMPOSITION FOR THE TREATMENT OR PROPHYLAXIS OF VIRAL INFECTIONS**

(71) Applicant: Kelin, Leonid Valerievich, Moscow 119192 (RU)
(72) Inventor: Kelin, Leonid Valerievich, Moscow 119192 (RU)
(74) Representative: KASTEL Patentanwälte PartG mbB
(86) International application number: PCT/RU2016/000623
(87) International publication number: WO 2018/048322

(57) **Abstract**

The invention is an antiviral drug. The invention offers a composition for treatment of livestock containing enzymes extracted from the bacterial strain Serratia marcescens M-10, deposited in the Russian National Collection of Microogranisms under No. VKM B-2931D.

## Description

### FIELD OF THE INVENTION

The invention relates to veterinary medicine, agriculture, pharmaceutics and biotechnology, and more precisely - to prophylaxis and treatment of viral diseases in livestock and bees.

### BACKGROUND OF THE INVENTION

Intensification and industrialization of livestock and poultry farming is often accompanied by the spread of viral diseases in animals. It is well known that the pathogens causing respiratory diseases are mainly influenza and parainfluenza viruses, infectious rhinotracheitis virus, adenoviruses, enteroviruses, herpes viruses and others, while bacterial infection is secondary. Respiratory infections cause significant of economic damage to livestock and poultry farming, reducing economic efficiency of farms by 30÷40% or more.

In beekeeping losses caused by viral diseases (acute and chronic paralysis, filamentovirosis, sacbrood disease, egyptovirosis) account for 20÷80%.

However, the range of products available for prophylaxis and treatment of viral diseases is very limited. Development of treatments and preventatives for viral diseases in livestock and poultry farming remains a topical problem.

### INVENTION DISCLOSURE

This invention aims to develop another contact action antiviral agent which may be used as an alternative to existing similar products (refer to Russian Federation patents No. 2423136 and No.2038776) and may have certain advantages due to the differences in the nature and origin of the enzymes. The advantages include elimination of allergic reactions and intolerance to existing products, more intensive action against certain kinds of viruses, overcoming resistance of the viruses to existing drugs, longer lasting action, potentiation of other treatments when used in combination.

Thus, according to the first aspect of the present invention we are offering a strain of bacteria *Serratia marcescens M-10,* deposited in the Russian National Collection of Microorganisms under No. VKM B-2931D.

According to the second aspect of the present invention we are offering enzymes extracted from that bacterial strain.

The enzymes indicated may include:
- endonuclease, and/or
- ribonuclease, and/or
- desoxyribonuclease, and/or
- lipase, and/or
- protease.

The enzymes may be extracted from culture fluid and/or whole-cell homogenate in various ways, for instance via exclusion chromatography.

According to the third aspect of the present invention we are offering a composition for treatment or prophylaxis of viral infections which contains an effective amount of item 3 endonuclease, or endonuclease whose sequence of aminoacid residuals is at least 95% homologous.

The composition, characterized in general terms, is further explained through several examples of especially preferable embodiments which provide additional advantages.

In one specific embodiment of the composition the aforementioned effective amount of endonuclease is 150÷300mg with activity level of 150÷400 ths. activity units (a.u.) per 1g.

In another embodiment, the composition additionally contains ribonuclease (RNAse) with activity level of 500÷4500 a.u., preferably extracted from the bacterial strain mentioned above, in the amount of 2÷4mg per 1g.

In another embodiment, the composition additionally includes desoxyribonuclease (DNAse) with activity level of 1000÷1800 a.u., preferably extracted from the bacterial strain mentioned above, in the amount of 2÷5mg per 1g.

In another embodiment, the composition additionally contains protease with activity level of 0.01÷0.06 a.u., preferably extracted from the bacterial strain described above, in the amount of 0.1÷0.3mg per 1g.

In another embodiment, the composition additionally contains lipase with activity level of 0.5÷4.0 mE a.u., preferably extracted from the bacterial strain indicated above, in the amount of 0.05÷0.2mg per 1g.

In another embodiment, the composition additionally contains 300÷620mg of magnesium sulphate per 1g.

In another embodiment, the composition additionally contains 100÷500mg dextrane, preferably around 180mg per 1g.

In another embodiment, the composition contains enzymes according to any of the items 2-7 in the amount of 150÷350mg per 1g, magnesium sulphate in the amount of 0.3÷0.62g and, optionally, inactive ingredients such as dextrane comprising the rest.

In another embodiment, the composition contains the following components per 1g:
- endonuclease with bacterial activity level of 150÷400 ths. a.u., 150÷350mg;
- RNAse with activity level of 500÷4500 a.u., 2÷4mg;
- DNAse with activity level of 1000÷8100 a.u., 2÷5mg;
- protease with activity level of 0.01÷0.06 a.u., 0.1÷0.3mg;
- lipase with activity level of 0.5÷4 mE, 0.05÷0.2mg,
- magnesium sulphate, 300÷620mg,
- dextrane - the rest.

In another embodiment, the composition is obtained through lyophilization of optionally buffered solution.

In another embodiment of the composition cryoprotectors selected from a group including dextrose, sorbitol, mannitol, mannose, sucrose and their mixture are added before lyophilization.

According to the fourth aspect of the present invention we suggest applying the composition according to any of the methods described in items 9-20, in form of a solution for prophylaxis and treatment of viral diseases in livestock.

In one particular application of the compositon the aforementioned solution is prepared by dissolving a dry mixture (not containing magnesium sulphate) in a solution that contains magnesium sulphate, or by adding magnesium sulphate to the solution that does not contain magnesium sulphate.

In another application the described solution is added to the feed as a supplement.

In another application the described solution is applied topically, intranasally or in form of aerosol.

In another application of the composition the viral disease in question is viral bronchopneumonia of calves and foals, preferably selected from a group including rhinotracheitis, respiratory syncytial virus infection, parainfluenza-3 virus infection and infection with adenoviruses type 1 and 2.

In another application of the composition the viral diseases in question are viral avian diseases, preferably selected from a group including infectious laryngotracheitis, Newcastle disease, enteroviral infections.

Another application of the composition is prophylactic usage to reduce viral load in newborn chicks.

In another application of the composition the viral diseases in question are viral apian diseases, preferably selected from a group including acute and chronic paralysis, filamentovirosis, sacbrood disease and egyptovirosis.

Another application of the composition is spring and summer stimulation of bee colonies.

Application of the present invention is further explained using several specific implementations which should not be understood as limiting the extent of patent protection.

### APPLICATION OF THE INVENTION

The composition that was selected for testing contains, per 1g of dry matter, an enzyme complex extracted from Serratia marcescens M-10 strain (deposited in Russian National Collection of Microorganisms under No. VKM B-2931D) and including endonuclease, ribonuclease, desoxyribonuclease, protease and lipase in the amount of 150÷350mg, magnesium sulphate in the amount of 0,3-0,62g, as well as inactive ingredients - dextrane in the amount of 180mg.

Endonuclease is the most active antiviral component of the composition, while other enzymes enhance its action by destroying the lipid membranes of the virus and/or capsids and/or accelerating further degradation of nucleic acids.

The endonuclease itself is an extracellular DNA/RNA-nonspecific enzyme, so it's active against both DNA and RNA viruses.

The enzyme complex used in test samples is obtained by cultivating the aforementioned Serratia marcescens M-10 strain with further precipitation of enzymes and drying of the precipitate.

What separates this endonuclease from the analogous enzymes is its extremely high hydrolytic activity towards DNA and RNA. This endonuclease catalyzes hydrolytic decomposition of DNA and RNA between 5'-phosphate and 3'-oxygen in sugars in the presence of Mg2 ⁺ions. This enzyme can decompose both one - and two-stranded substrates with equal efficiency. Antiviral activity of the enzyme is caused by its ability to hydrolyze nucleic acids via endonucleic mechanism to mono-, di-, tri-, tetra-and oligonucleotides. When subjected to the enzyme, viral DNA and RNA lose their ability to serve as a matrix for nucleic acid and protein synthesis.

The endonuclease described above is synthesized in the form of protein predecessor 266 aminoasid residuals in length and a signal peptide consisting of the first 21 aminoacid residuals. Two main isoforms of the enzyme with similar biological properties have been identified - Sm1 and Sm2. Sm1 lacks the first three N-terminal aminoacid residuals. Sm2 (molecular weight of 26.7 kD) consists of 245 aminoacid residuals and is a result of processing - detachment of the signal peptide.

During sequence analysis it was established that the endonuclease contains sequences homologous to the sequences of the following loci of sequenced genome of *Serratia marcescens subsp. marcescens Db11* and their parts: SMDB11_RS07495, SMDB11_RS05280, SMDB11_RS07940, SMDB11_RS07935, SMDB11_RS11830 (GENE DATABASE, http://ncbi.nlm.nih.gov).

The active center of the enzyme includes four aminoacid residuals, namely Arg57, His89, Asn119 and Glu127, located immediately next to each other. His89 takes part in substrate binding and is deprotonated, thus serving as a basis. Arg57 and Arg87 take part in catalysis. Arg57 takes part in positioning and polarization of phosphatefissile phosphodiester bond and in transition state stabilization. Asn119 contributes to metal ions binding. Glu127 takes part in hydrolysis. The site of magnesium ions binding is located between the spiral (aminoacid residuals 116-135) and the loop made by 50 to 114 residuals. A mutation in any of the aminoacid residuals, especially in His89, can cause a dramatic decrease in enzyme activity. Replacement of Asn119 and Arg57 aminoacids also reduces enzyme activity.

The aforementioned ribonuclease (RNAase) is nuclease H which catalyzes RNA degradation. RNAase H is a nonspecific endonuclease and it catalyzes RNA decomposition through hydrolysis in the presence of a trapped bivalent metal ion. 5 '-phosphorylated product is formed as a result of ribonuclease H influence.

The aforementioned desoxyribonuclease (DNAase) is a nuclease which catalyzes hydrolytic decomposition of polynucleotid single-stranded or double-stranded DNA, forming separate nucleotides and oligonucleotides with terminal 5'-phosphate and a free hydroxyl group on 3'-end.

The aforementioned protease is a hydrolytic enzyme which breaks down the peptide bond between aminoacids. The neutral protease content in the test sample of the composition was 0.01-0.06 a.u./g, and alkaline protease content was 0.015-0.04 a.u./g. The amount of enzyme needed to bring the protein into a state where it is not non-precipitable by trichloroacetic acid in the amount corresponding to 1 micromole of tyrosine in 1 min at 30°C is accepted as a unit of general proteolytic activity. Proteolytic activity of neutral proteases is determined at 7.0 pH of the reaction mixture and at 9.0 pH for alkaline proteases.

Lipase in the aforementioned strain catalyzes hydrolysis of insoluble lipid substrates. Lipase content in 1g of the test sample was 0.5-4 mE. The amount of enzyme produced after the sample is treated with the standard TNB buffer for 1 min at 37°C is accepted as one activity unit of lipase.

Magnesium sulphate in the test sample increases activity of the enzymes that break phosphodiester bonds.

Dextrane acts as stabilizer and filler, its content in the composition can largely vary. It is preferable to use dextrane with molecular weight of 40-80 kD.

The composition can also contain cryoprotectors and buffers.

The test sample used in examples 1-7 was obtained from a composition which contained the following components per 1g of dry matter:
- endonuclease with bacterial activity level of 150-400 ths. a.u., 150÷350mg,
- RNAse with activity level of 500÷4500 a.u., 2÷4mg,
- DNAse with activity level of 1000÷8100 a.u., 2÷5mg,
- protease with activity level of 0.01÷0.06 a.u., 0.1÷0.3mg,
- lipase with activity level of 0.5÷4 mE, 0.05÷0.2mg,
- dextrane - the remainder.

The sample is a powder with color ranging from white to light brown, very soluble in water, almost insoluble in organic solvents.

In the present text an activity unit of endonuclease (a.u.) is the amount of the enzyme which causes increase of optical density of the acid-soluble RNA or DNA hydrolysates by 1 optical unit at wavelength of 260 nanometers for 20 minutes at 37 °C.

It was established that the composition shows antiviral activity both *in vitro* and *in vivo* and in particular inhibits replication of vesicular stomatitis and variolovaccine viruses in a culture of chicken fibroblast cells.

The composition is non-toxic for humans, animals and plants in almost any reasonable range of doses and spontaneously deactivates in the environment.

### Example 1.

### Influence on reproduction of the Newcastle disease virus in vitro

Infectious activity of Newcastle disease virus cultivated in the presence of the test sample in concentration of 50 a.u. and 100 a.u. was 9.0 1g LD50/cm³ and 9.5 1g LD50/cm³, respectively.

Infectious activity of control virus was 9.9 1g LD50/cm³.

Therefore, the composition in the concentration of 100 a.u. reduces infectious activity of the virus 2.5 times, and in the concentration of 50 a.u. - eightfold.

### Example 2.

### Evaluation of antiviral activity toward infectious bird laryngotracheitis virus (ILT) on chicks

50.000 a.u. of dry sample without magnesium sulphate is dissolved in 300ml of room temperature boiled water. 0.62g of magnesium sulphate is then added to the solution.

Chicks are treated in a 2m³ aerosol chamber according to the following procedure:
- treatment with the sample 1 hour prior to infection,
- 1 hour after infection,
- 24 hours after infection,
- 48 hours after infection.

Chicks were infected using aerosol method, exposure time was 20 minutes.

Dynamics of disease and mortality are shown in table 1.

The results show that aerosol treatment of chicks with the solution containing the recommended dose of enzyme complex, if the recommended procedure is followed, protects against infection with ILT virus in the amount of 10 infectious doses per cm³ (ID/cm³). No disease was observed in the birds that underwent treatment, while 20% of untreated birds showed signs of disease. When chicks were exposed to 100 ID/cm³ of ILT virus the rate of infection in experimental group was two times lower compared to the control group - 40% and 80%, respectively.

Treatment also protects poultry from death: no mortality was observed in the experimental group, compared to 25% mortality in the control group.

### Example 3.

### Prophylaxis of avian ILT

Industrial tests of the composition's effectiveness against the avian ILT virus were carried out in a 1650m² enclosure, results are shown in table 2.

15ml of treatment solution per 1m² is prepared. The composition is applied in the form of fine spray using a cold fogger. The spray layer covered the area of at least 40cm off the floor, particle size - 5÷15 micron.

As a result of using the treatment solution for prophylaxis a 2.2 times mortality decrease was observed, as well as a 2.95% increase in stock livability. An increase in average daily gain and commercial yield was also observed.

### Example 4.

### Activity toward infectious bovine rhinotracheitis (IBR) (belongs to the Herpesviridae family) in vitro

Infectious activity of the IBR virus cultivated in the presence of the test sample in concentration of 50 a.u. and 100 a.u. was 6.25 1g TCD50/cm³ and 6.0 1g TCD50/cm³, respectively.

Infectious activity of the control virus was 6.75 1g TCD50/cm³.

Therefore, the composition in concentration of 100 a.u. reduces infectious activity of the IBR virus 5÷6 times, and in concentration of 50 a.u. - threefold.

### Example 5.

### Activity toward IBR in vivo

0.5÷2 month old calves were treated with the composition intranasally, twice with a 7÷10 day interval.

The composition solution, when introduced to the nasal cavity, exhibits prophylactic and therapeutic action against acute disease. No mortality was observed in the experimental groups (mortality in the control groups was, on average, 5%). 98% of the calves in the experimental groups recovered, compared to only 12% in control groups.

Therefore, the test sample is highly effective against IBR virus.

### Example 6.

### Investigation into the feasibility of prophylaxis and treatment of viral respiratory diseases in foals

Studies were carried out at an industrial heifer breeding complex OAO Plemzavod "Uchkhoz Tulinskoye", on Soviet Heavy Draft foals 20÷60 days of age.

Prophylactic action of the test sample was tested on 49 foals. 25 of them received the sample intranasally in a single 2000 a.u. dose, while 24 foals received the same dosage twice with a 10 day interval.

After administration of this dose of the drug the general condition of the animals did not change: body temperature, heart rate and respiratory rate remained within normal limits.

Control group animals (23 foals) did not receive the drug. The foals were observed for 2 months.

In that time period among 49 foals that received endonuclease 7 showed signs of respiratory disease (14.3%). They had a mild form of the disease and all of them recovered after administration of regular treatment. In the control group 17 foals out of 23 had bronchopneumonia (74%). 2 of them were severely ill, they were culled and sent to a meat-packing plant (table 3).

Therefore, a single intranasal administration of a 2000 a.u. dose of the drug to 20÷60 day old foals reduces the incidence of respiratory disease more than fivefold and also reduces severity of illness, which prevents forced slaughter.

### Example 7.

### Investigation into the feasibility of prophylaxis and treatment of viral respiratory apian diseases

Bee colonies were kept in 16-frame Dadant-Blatt hives. 4 groups of bee colonies were used in the experiment, each comprising 10 colonies:
Group 1 - received treatment via aerosol spraying, each beeway was treated 3 times with a 7 day interval;
Group 2 - the drug was dissolved in sugar syrup and fed to the bees in one day, 3 times with a 7 day interval;
Group 3 - treated by water aerosol, 3 times with a 7 day interval;
Group 4 - control, no treatment.

The results of testing the prophylactic and treatment action of the composition are shown in table 4.

The proposed composition containing an enzyme complex demonstrates effectiveness against viral diseases in bee colonies.

The application of the composition via aerosol and with sugar syrup in spring time for prophylaxis and treatment of viral diseases in bee colonies and for stimulation of bee colonies' develioment promotes more rapid strengthening of the colonies and larger brood, which eventually leads to higher honey and wax production.

**Table 1.**

| | | **Dynamics of disease incidence and mortality in chicks after infection with a virulent ILT virus** | | | | | | | | | | |
|---|---|---|---|---|---|---|---|---|---|---|---|---|
| Group, infection dose | Subgroup | Days after infection | | | | | | | | | | |
| | | 0 | 1 | 2 | 3 | 4 | 5 | 6 | 7 | 8 | 9 | 10 |
| **1** | 1 | 0/10 | 0/10 | 0/10 | 0/10 | 0/10 | 0/10 | 0/10 | 0/10 | 0/10 | 0/10 | 0/10 |
| 10 ID/cm³ | 2 | 0/10 | 0/1 | 0/10 | 0/10 | 2i/10 | 2i/10 | 2i/10 | 2i/10 | 2i/10 | 2i/10 | 2i/10 |
| **2** | 1 | 0/10 | 0/10 | 0/10 | 0/10 | 0/10 | 0/10 | 4i/10 | 4i/10 | 4i/10 | 4i/10 | 4i/10 |
| 100 ID/cm³ | 2 | 0/10 | 0/10 | 0/10 | 0/10 | 6i/10 | 8i/10 | 8i/10 | 8i/10 | 8i/10 | 2d/5i/10 | 2d/5i/10 |
| **3** Control | not infected | 0/10 | 0/10 | 0/10 | 0/10 | 0/10 | 0/10 | 0/10 | 0/10 | 0/10 | 0/10 | 0/10 |

| | | | | | | | | | | | | |
|---|---|---|---|---|---|---|---|---|---|---|---|---|
| Notes: in the numerator: d-dead, i-ill; in the denominator: 10 (total number or chicks in a group); ID -infection dose. | | | | | | | | | | | | |

**Table 2.**

| **Comparison of results in control and test poultry yards housing broiler stock** | | |
|---|---|---|
| **Indicator** | **Control** | **Test** |
| Set (heads) | 34455 | 35620 |
| Mortality per breeding cycle (heads) | 1837 | 857 |
| Viability % | 94.64 | 97.57 |
| Feeding days | 39.4 | 40.5 |
| Total weight gain (kg) | 71695 | 83197 |
| Daily average weight gain (g) | 54.91 | 59.34 |
| Slaughtered (heads) | 32608 | 34757 |
| Live weight before slaughter (kg) | 70547 | 83534.7 |
| Slaughter weight (kg) | 51693 | 63013.5 |
| Meat yield (%) | 73.27 | 75.43 |
| Offal yield (%) | 11.26 | 10.05 |
| Total production (kg) | 59636 | 71405.4 |
| Production yield (%) | 84.5 | 85.48 |
| European broiler index | 324 | 362.55 |

**Table 3.**

| **Results of testing the prophylactic action of the composition containing an enzyme complex against respiratory diseases in foals** | | | | | |
|---|---|---|---|---|---|
| **Group** | **Number of foals, heads** | **Foals that were ill** | | **Culled foals** | |
| | | **heads** | **%** | **heads** | **%** |
| **Test** | 49 | 7 | 14.28 | - | - |
| **Control** | 23 | 17 | 74 | 2 | 8.7 |

**Table 4.**

| **Results of using the composition containing an enzyme complex to treat viral diseases in bees and to stimulate bee colonies** | |
|---|---|
| **Application** | **Result** |
| For prophylaxis and treatment, aerosol spraying | Group 1 - all bee colonies healthy |
| | Control (Group 4) showed signs of viral disease (sacbrood disease) in 5 colonies |
| For prophylaxis and treatment, with sugar syrup | Group 2 - all bee colonies healthy |
| Stimulation of bee colonies, with sugar syrup | During testing the bee colonies belonging to Group 2 surpassed the performance of control group colonies by 20.2% on colony strength, by 58.8% on brood, by 30.3% on honey production, by 50% on beeswax production. |
| Stimulation of bee colonies, aerosol spraying | During testing the bee colonies belonging to Group 1 surpassed the performance of control group colonies by 26.2% on colony strength, by 64.2% on brood, by 35.6% on honey production, by 50% on beeswax production. |

## Claims

1. Bacterial strain *Serratia marcescens M-10*, deposited in the Russian National Collection of Microorganisms under No.VKM B-2931D.

2. Enzymes extracted from the bacterial strain in item 1.

3. Enzymes according to item 2, containing endonuclease.

4. Enzymes according to item 2, containing RNAse.

5. Enzymes according to item 2, containing DNAse.

6. Enzymes according to item 2, containing lipase.

7. Enzymes according to item 2, containing protease.

8. Enzymes according to item 2, extracted individually via exclusion chromatography.

9. Composition for treatment or prophylaxis of viral infections, containing an effective amount of endonuclease from item 3 or endonuclease whose aminoacid residuals sequence is at least 95% homologous to it.

10. Composition according to item 9, containing an efficient amount of endonuclease specified as 150÷300mg with activity level of 150÷400 ths. activity units (a.u.) per 1g.

11. Composition according to item 9 additionally containing ribonuclease (RNAse) with activity level of 500÷4500 a.u., preferably according to item 4, in the amount of 2÷4mg per 1g.

12. Composition according to item 9 additionally containing desoxyyribonuclease (DNAse) with activity level of 1000÷8100 a.u., preferably according to item 5, in the amount of 2÷5mg per 1g.

13. Composition according to item 9 additionally containing protease with activity level of 0.01÷0.06 a.u., preferably according to item 6, in the amount of 0.1÷0.3mg per 1g.

14. Composition according to item 9 additionally containing lipase with activity level of 0.5÷4.0 mE a.u., preferably according to item 7, in the amount of 0.05÷0.2mg per 1g.

15. Composition according to item 9 additionally containing magnesium sulphate in the amount of 300÷620mg per 1g.

16. Composition according to item 9 additionally containing 100÷500mg dextrane, preferably around 180mg per 1g.

17. Composition according to item 9 including any of the enzymes indicated in items 2-7 in the amount of 150÷350mg per 1g, 0.3÷0.62mg magnesium sulphate and, optionally, inactive ingredients such as dextrane comprising the rest.

18. Composition according to item 9 containing the following components per 1g:
- endonuclease with bacterial activity level of 150÷400 ths. a.u., 150÷350mg;
- RNAse with activity level of 500÷4500 a.u., 2÷4mg;
- DNAse with activity level of 1000÷8100 a.u., 2÷5mg;
- protease with activity level of 0.01÷0.06 a.u., 0.1÷0.3mg;
- lipase with activity level of 0.5÷4 mE, 0.05÷0.2mg,
- magnesium sulphate, 300÷620mg,
- dextrane - the remainder.

19. Composition according to item 9 obtained through lyophilization of optionally buffered solution.

20. Composition according to item 18 to which cryoprotectors selected from a group including dextrose, sorbitol, mannitol, mannose, sucrose and their mixture were added before lyophilization.

21. Application of the composition according to any of the methods described in items 9-20, in form of a solution for prophylaxis and treatment of viral diseases in livestock.

22. Application according to item 21 in which the solution is prepared by dissolving a dry mixture (not containing magnesium sulphate) in a solution that contains magnesium sulphate, or by adding magnesium sulphate to the solution that does not contain magnesium sulphate.

23. Application according to item 21 in which the composition solution is added to livestock feed as a supplement.

24. Application according to item 21 in which the described solution is applied topically, intranasally or in form of aerosol.

25. Application according to item 21 in which the viral disease in question is viral bronchopneumonia of calves and foals, preferably selected from a group including rhinotracheitis, respiratory syncytial virus infection, parainfluenza-3 virus infection and infection with adenoviruses 1 and 2.

26. Application according to item 21 the viral diseases in question are viral avian diseases, preferably selected from a group including infectious laryngotracheitis, Newcastle disease, enteroviral infections.

27. Application according to item 21 in which the composition is used as a prophylactic to reduce viral load in newborn chicks.

28. Application according to item 21 in which the viral diseases in question are viral diseases of bees, preferably selected from a group including acute and chronic paralysis, filamentovirosis, sacbrood disease and egyptovirosis.

29. Application according to item 21 in which the composition is used for spring and summer stimulation of bee colonies.
